# EUROPEAN PATENT SPECIFICATION

(11) **EP 3 612 142 B1**
(45) Date of publication and mention of the grant of the patent: **02.07.2025**
(21) Application number: 18721446.5
(22) Date of filing: 19.04.2018
(51) Int. Cl.: A61F 7/02, A61F 7/08, A61F 9/04

(54) **MEDICAL DEVICE**
MEDIZINISCHE VORRICHTUNG
DISPOSITIF MÉDICAL

(30) Priority: 20.04.2017 GB 201706302; 08.03.2018 GB 201803705
(43) Date of publication of application: 26.02.2020
(73) Proprietor: Grant, Susan, Huddersfield, West Yorkshire HD8 8QH (GB); Wymer, Adam, Huddersfield, West Yorkshire HD8 8QH (GB); Wymer, Sam, Huddersfield, West Yorkshire HD8 8QH (GB)
(72) Inventor: Grant, Susan, Huddersfield, West Yorkshire HD8 8QH (GB); Wymer, Adam, Huddersfield, West Yorkshire HD8 8QH (GB); Wymer, Sam, Huddersfield, West Yorkshire HD8 8QH (GB)
(74) Representative: Petty, Catrin Helen
(86) International application number: PCT/GB2018/051033
(87) International publication number: WO 2018/193262

(56) References cited:
- KR-A- 20070 021 645
- KR-A- 20120 123 926
- KR-A- 20120 123 926
- KR-U- 20100 002 818
- KR-U- 20100 002 818
- US-A1- 2005 119 629
- US-A1- 2005 119 629
- US-A1- 2009 287 283
- US-A1- 2009 287 283
- US-A1- 2009 293 882
- US-A1- 2013 040 526
- US-A1- 2013 172 829
- US-A1- 2013 172 829
- US-A1- 2014 330 222
- US-A1- 2014 330 222
- US-A1- 2015 320 589
- US-A1- 2015 320 589
- US-B1- 9 095 566

## Description

The invention relates to medical devices, and particularly, although not exclusively, to medical devices for treating common conditions, injuries or ailments suffered in or on the eyes, and surrounding areas.

The meibomian glands (or tarsal glands) are a special kind of sebaceous gland at the rim of the eyelids inside the tarsal plate, responsible for the supply of meibum, an oily substance that prevents evaporation of the eye's tear film. Meibum prevents tear spillage onto the cheek, trapping tears between the oiled edge and the eyeball, and makes the closed lids airtight. Dysfunctional meibomian glands often cause dry eyes, one of the more common eye conditions. They may also cause blepharitis, as the dry eyeball rubs off small pieces of skin from the eyelid, which may get infected. Inflammation of the meibomian glands (also known as meibomitis, meibomian gland dysfunction, or posterior blepharitis) causes the glands to be obstructed by thick waxy secretions. Besides leading to dry eyes, the obstructions can be degraded by bacterial lipases, resulting in the formation of free fatty acids, which irritate the eyes and sometimes cause punctate keratopathy.

One treatment for such common eye conditions which is currently available comprises a pouch filled with a temperature control means. The pouch is disposed within a removable cover which, in use, contacts a subject's eyes and a surrounding area thereof and the temperature control means modulate the temperature of the cover, and thereby heats the subject's eyes and the surrounding area. After the heat treatment has been applied the subject can massage the area around their eyes and wipe away any debris. The device, when used in this method, treats, prevents or ameliorates the eye condition.

However, the inventors have found that in use bacteria from the eye can be transferred to the removable cover. The bacteria can then re-infect the eyes of a user on subsequent use of the cover. Furthermore, the temperature of the temperature control means can be affected by the external environment leading to variations in temperature.

US 2005/119629 A1 describes a pad for treating eye conditions. The pad comprises a multipart container having an impermeable outer membrane sized to fit generally within the peri-orbital region and sufficiently flexible to mold to the eye. The pad further comprises a first chemical in a first storage area in the multipart container and a second chemical in a second storage area in the multipart container. The first and second chemicals are selected to have an exothermic reaction when mixed for producing a temperature suitable for treating eye conditions.

US 2014/330222 A1 describes an article for therapy and treatment of an eye. The article includes a backing for application to the eye. The backing includes at least one receiver positioned to align with the eye. The article also includes at least one pod that is detachably secured within the at least one receiver. The at least one pod includes material for delivering therapy and treatment to the eye.

US 2013/172829 A1 describes an apparatus for treating dry eye. The apparatus comprises a patch or strip affixed to the skin of the upper and/or lower eyelids to deliver heat or other forms of energy, pressure, drugs, moisture, etc. (alone or in combination) to the one or more meibomian glands contained within the underlying skin. The treatment strip or strips include one or more strips configured to adhere to an underlying region of skin in proximity to one or both eyes of a subject such that the one or more strips allow for the subject to blink naturally without restriction from the one or more patches.

US 2009/287283 A1 describes a thermally adjustable compress assembly. The compress assembly includes a thermally adjustable gel pack and an external frame positionable against the outwardly facing surface of the gel pack. The external frame can be passively positionable against the gel pack to apply backward pressure against the gel pack during use or can be actively attached to the gel pack to provide additional support to at least a portion of the gravitational weight of the gel pack during use.

KR 2012 0123926 A describes an eye compress comprising a cover and fillers. The cover is formed by facing a pair of cotton sheets and sewing the edges of the cotton sheets. The fillers are put into the cover and are made of stone capable of irradiating far infrared rays. The cover is coated with one or more selected from silver, gold, and platinum.

US 2015/320589 A1 describes a heating tool which may be attached to the skin of a user, may warm the skin and supply oil thereto. The heating tool includes an adhesive layer which has a specific heat of less than 3 J/g·K at 20° C. The adhesive layer includes a base and oil with a specific heat of less than 3 J/g·K at 20° C and a boiling point of 100° C or higher at 1 atmospheric pressure, and the oil is contained in an amount of 30 to 90 wt % in the adhesive layer.

KR 2010 0002818 U describes a cooling eye compress. The compress comprises a phase change material.

KR 2007 0021645 A describes a multifunctional eye bandage. The eye bandage comprises silver nanoparticles which have antibacterial and deodorising properties. KR 2007 0021645 A explains that silver nanoparticles and tourmaline, a material which emits far infra-red rays, can be contained in that material out of which the eye patch is made.

US 2013/040526 A1 is concerned with building materials and other construction components. **In** particular, US 2013/040526 A1 discloses a material for energy management and peak energy reduction in a building structure.

US 2009/293882 A1 relates to antimicrobial compositions which may be used with medical devices, such as catheters and implants.

US 9,095,566 B1 relates to compositions comprising silver nanoparticles for treating eyelash and eyelid margin infections.

The present invention arises from the inventor's work in attempting to overcome the problems associated with the prior art.

Hence, according to a first aspect of the invention, there is provided a medical device for treating, preventing or ameliorating an eye condition, the device comprising a cover, at least a portion of which comprises an antimicrobial agent and is at least partially filled with temperature control means, wherein, the antimicrobial agent comprises silver, or a pharmacologically acceptable salt thereof, and an oxide, wherein the oxide is selected from the group consisting of copper oxide, iron oxide, zinc oxide, titanium dioxide and silica dioxide, and, in use, the cover is arranged to contact a subject's eye and/or a surrounding area thereof, and wherein the temperature control means is configured to modulate the temperature of the cover to thereby treat, prevent or ameliorate the eye condition.

Advantageously, and preferably, the antimicrobial agent is configured to kill unwanted micro-organisms (e.g. yeast and/or bacteria) that comes into contact with the cover, and thereby prevents infection and/or reinfection of the subject's eye. Preferably, the device is used to prevent the eye condition from occurring in the first place or reoccurring, because it improves eyelid hygiene by ensuring that the meibomian glands are kept clean. It achieves this by effectively dissolves debris and bacteria etc. present within the oily meibum produced by the meibomian glands. It will be appreciated that use of the medical device of the invention is the first step in eye/eyelid hygiene followed by massage of the eye and/or the surrounding area thereof, and removal of any debris. Effective use of the device of the invention, therefore, improves eye hygiene, and confidential trials have shown that use of such a device can effectively treat many eye conditions.

Preferably, the cover is made of a material which effectively dissipates heat to a user's eye and surrounding area, when the device is in use. A first side of the cover may be made of a first material, which dissipates heat when the device is in use, at a greater rate than that of a second side of the cover made of a second material. For example, the first material may comprise synthetic or naturally occurring fibres. Preferably, the first material comprises naturally occurring fibres. Examples of the first material include cotton, silk, satin, or polyester. Silk is preferred.

The second material may comprise velvet, velour or suede. However, in a preferred embodiment, the second material may comprise a phase change material (PCM).

Advantageously, the PCM thermally regulates the temperature of the device, thereby ensuring a roughly consistent temperature is delivered to the eyes of the subject. Furthermore, the PCM reduces loss of heat to the environment, thereby allowing the temperature to be delivered for an extended period of time.

The first material may be configured to dissipate heat, when the device is in use, at a greater rate than that of the second side of the cover made of the second material. For example, the first material may comprise synthetic or naturally occurring fibres. Preferably, the first material comprises naturally occurring fibres. Examples of the first material include cotton, silk, satin, or polyester. Silk is preferred.

The second material may comprise fibres with the PCM located therein. However, in a preferred embodiment, the second material comprises a fabric with a layer of the PCM disposed thereon. Preferably, the layer of the PCM is disposed on an internal and/or external surface of the fabric. More preferably, the layer of the PCM is disposed on the internal surface of the fabric. The fabric may comprise synthetic or naturally occurring fibres.

Preferably, the device comprises a pouch disposed within the cover and is at least partially filled with the temperature control means. Preferably, the cover is removable. Accordingly, the pouch may be removably disposed in the cover.

It may be appreciated that the internal surface is the surface that is disposed substantially adjacent to, and preferably contacts, the temperature control means or, in embodiments where it is present, the pouch.

Preferably, the antimicrobial agent is configured to contact a person's eye without causing damage or irritation thereto. The antimicrobial agent may comprise a cationic surfactant. The cationic surfactant preferably comprises benzalkonium chloride (BKC). Advantageously, BKC is used in eye drops and contact lens solutions, and may safely contact a person's eye. Alternatively, or additionally, the antimicrobial agent may comprise a polymer. Preferably, the polymer comprises a biguanide polymer, more preferably polyaminopropyl biguanide (PAPB).

Advantageously, a polymer would not migrate into a user's skin.

The polymer may have an average molecular weight between 10,000 and 30,000, more preferably between 13,000 and 23,000.

The antimicrobial agent comprises silver or a pharmaceutically acceptable salt thereof.

Pharmaceutically acceptable salts include any salt which retains its biological properties and which is not toxic or otherwise undesirable for pharmaceutical use. The pharmaceutically acceptable salt may be derived from a variety of organic and inorganic counter-ions well known in the art.

Accordingly, the salt may be a halide, a sulphate or a nitrate.

Preferably, the antimicrobial agent may comprise a silver salt. The silver salt may be selected from the group consisting of silver sulphate, silver nitrate, silver chloride, silver sodium zirconium hydrogen phosphate and silver sulfadiazine.

Preferably, the oxide is titanium dioxide.

Preferably, the antimicrobial agent comprises a silver salt and titanium dioxide. Preferably the silver salt is silver chloride.

Preferably, the weight ratio of the metal, or the salt thereof, to the oxide is between 20:1 and 1:50, more preferably between 5:1 and 1:20 or between 2:1 and 1:10, and most preferably between 1:1 and 1:8 or between 1:2 and 1:6.

Preferably, an external surface of the first side of the cover comprises the antimicrobial agent. It may be appreciated that, the first side of the cover, and more specifically external surface thereof, is configured to contact the user's eye. Alternatively or additionally, an internal surface of the first side of the cover may comprise the antimicrobial agent.

Preferably, the concentration of the antimicrobial agent is at least 1 x 10⁻¹¹ mol/cm², more preferably at least 1 x 10⁻¹⁰ mol/cm² or at least 5 x 10⁻¹⁰ mol/cm², and most preferably at least 1 x 10⁻⁹ mol/cm², at least 2 x 10⁻⁹ mol/cm² or at least 3 x 10⁻⁹ mol/cm². Preferably, the concentration of the antimicrobial agent is less than 1 x 10⁻⁴ mol/cm², more preferably less than 1 x 10⁻⁵ mol/cm² or less than 1 x 10⁻⁶ mol/cm², and most preferably less than 1 x 10⁻⁷ mol/cm², less than 5 x 10⁻⁸ mol/cm² or less than 1 x 10⁻⁸ mol/cm². Preferably, the concentration of the antimicrobial agent is between 1 x 10⁻¹¹ mol/cm² and 1 x 10⁴ mol/cm², more preferably between 1 x 10⁻¹⁰ mol/cm² and 1 x 10⁻⁵ mol/cm² or between 5 x 10⁻¹⁰ mol/cm² and 1 x 10⁻⁶ mol/cm², and most preferably between 1 x 10⁻⁹ mol/cm² and 1 x 10⁻⁷ mol/cm², between 2 x 10⁻⁹ mol/cm² and 5 x 10⁻⁸ mol/cm² or between 3 x 10⁻⁹ mol/cm² and 1 x 10⁻⁸ mol/cm².

Alternatively, or additionally, the fabric of the cover may comprise at least 0.00001 parts by weight of the antimicrobial agent. More preferably, the fabric of the cover comprises at least 0.00003 of the antimicrobial agent. Most preferably, the fabric of the cover comprises at least 0.00005 of the antimicrobial agent. Preferably, the fabric of the cover may comprise between 0.00001 and 0.001 parts by weight of the antimicrobial agent. More preferably, the fabric of the cover comprises between 0.00003 and 0.001 of the antimicrobial agent. Most preferably, the fabric of the cover comprises between 0.00005 and 0.0008 of the antimicrobial agent.

The pouch may also comprise the antimicrobial agent.

The device may be used to alleviate or treat a condition selected from the group of: styes, cysts, inflammation, dry eye disease, grittiness, irritation, itchy eyes (e.g. caused by hay fever), Meibomian Gland Dysfunction (MGD), Meibomian Gland Disease, Meibomian Gland Inspissation, chalazion, blepharitis, hordeolum, conjunctivitis, hay fever, headaches, tired or sore eyes, swelling/puffy eyes, allergies, and migraines.

Preferably, the temperature control means comprises grain or seed. For example, the temperature control means may be selected from a group of grain or seed consisting of: millet, rapeseed, canary seed, flax seed and wheat. In some embodiments, the temperature control means may comprise one or more type of seed or grain. Preferably, the temperature control means comprises at least two, at least three, or at least four different types of seed or grain. For example, the temperature control means may comprise a combination of wheat and flax (e.g. 50% of each), or alternatively a combination of rapeseed, flax and millet (e.g. about 1/3 of each), and so on. Any combination of the seeds and grains described herein is envisaged. In a preferred embodiment, however, the temperature control means comprises a combination of canary seed, rapeseed and millet seed, more preferably about 10-50% of each of canary seed, rapeseed and millet, and most preferably, a combination of about 25-35% of each of canary seed, rapeseed and millet.

In another embodiment, the temperature control means may comprise tourmaline beads. Tourmaline is a crystalline boron silicate mineral which can be produced into small beads.

In a most preferred embodiment, however, the temperature control means comprises silica beads or silica gel. The silica beads or gel may comprise an average diameter of at least 0.5mm, more preferably at least 2mm, and even more preferably at least 4mm. Preferably, the silica beads or gel may comprise an average diameter of less than 10mm, more preferably less than 8mm, and even more preferably less than 6mm. Preferably, the silica beads or gel may comprise an average diameter of between 0.5mm and 10mm, more preferably between 1mm and 7mm, and more preferably between 1mm and 6mm.

It will be appreciated that the optimum temperature of the eyelids does not exceed much more than 50°C. In one embodiment, the temperature control means is configured to be heated, preferably to above room temperature, i.e. above 21°C. Preferably, the temperature control means is configured to be heated to above 30°C, more preferably above 40°C, and even more preferably above 50°C. Preferably, the temperature control means is configured to be heated to below 70°C, more preferably below 60°C, and even more preferably below 55°C. Preferably, the temperature control means is configured to be heated to between 30 and 70°C, more preferably to between 40 and 60°C, even more preferably to between 42 and 47°C. The temperature control means may be heated by microwave energy, for example using a domestic microwave oven. Alternatively, it could be heated using a conventional oven. When contacted with the user, the heat is suitably transferred from the device to the user's eye or surrounding area.

Preferably, in this embodiment, the PCM has a transition temperature above room temperature, i.e. above 21°C. Preferably, the PCM has a transition temperature above 30°C, more preferably above 40°C, and even more preferably above 50°C. Preferably, the PCM has a transition temperature below 70°C, more preferably below 60°C, and even more preferably below 55°C. Preferably, the PCM has a transition temperature between 30 and 70°C, more preferably between 40 and 60°C, even more preferably between 42 and 47°C.

Preferably, the device is heated to treat any of the following conditions: styes, cysts, dry eye disease, grittiness, irritation, itchy eyes (e.g. caused by hay fever), Meibomian Gland Dysfunction (MGD), Meibomian Gland Disease, Meibomian Gland Inspissation, chalazion, blepharitis, and hordeolum.

In an embodiment in which the temperature control means comprises silica beads or silica gel and is configured to be heated, preferably the silica comprises an average diameter of between 0.5mm and 5mm, more preferably between 0.5mm and 4mm, and more preferably between 1mm and 3mm. Advantageously, the silica fits better around the glands in the eyelid.

Additionally, or alternatively, the temperature control means is arranged, in use, to be cooled, preferably to less than room temperature, i.e. less than 21°C. Preferably, the temperature control means is configured to be cooled to below 15°C, more preferably below 10°C, even more preferably below 5°C, and most preferably below 0°C. Preferably, the temperature control means is configured to be cooled to above -10°C, and more preferably above -5°C, even more preferably above -3°C. The temperature control means may be cooled by placing the device in a domestic fridge or freezer.

Preferably, in this embodiment, the PCM has a transition temperature which is less than room temperature, i.e. less than 21°C. Preferably, the PCM has a transition temperature which is less than 15°C, more preferably less than 10°C, even more preferably less than 5°C, and most preferably less than 0°C. Preferably, the PCM has a transition temperature which at least -10°C, and more preferably at least -5°C, even more preferably at least -3°C.

Preferably, the device is cooled to treat any of the following conditions: inflammation, irritation, itchy eyes (e.g. caused by hay fever), conjunctivitis, hay fever, headaches, tired or sore eyes, swelling/puffy eyes, allergies, and migraines.

When contacted with the user, the cooling energy is suitably transferred from the device to the user's eye or surrounding area. In this embodiment, the temperature control means may comprise seed or grain. Examples of grain, seed, tourmaline beads and/or silica beads or silica gel which may be used in the cooling embodiment are described above. However, in another embodiment, the temperature control means may comprise cooling crystals, which are configured to cool down when contacted with water.

In a preferred embodiment (when cooling is desired), the temperature control means comprises a cooling gel. The cooling gel preferably comprises silica beads or silica gel. The gel in the pouch may be put into a fridge or freezer, and when it is removed, the user preferably manipulates it to loosen it and make it pliable so that the pouch and cover mould around the eyes and surrounding areas.

In an embodiment in which the temperature control means comprises silica beads or gel and is configured to be cooled, preferably the silica comprises an average diameter of between 3mm and 8mm, more preferably between 4mm and 7mm, and more preferably between 5mm and 6mm. Advantageously, this size of silica retains the cold better than smaller beads.

Preferably, at least 30%, preferably at least 40%, more preferably at least 50%, and even more preferably at least 55%, and most preferably at least 60% of the volume of the cover or the pouch is filled with the temperature control means. Suitably, the temperature control means occupies less than 95%, preferably less than 90%, more preferably less than 80% of the volume of the cover or the pouch. It is preferred that the temperature control means occupy between about 40% and 95%, more preferably between about 50% and 80% of the volume of the space, most preferably between about 55% and 70% of the volume of the space.

Advantageously, this allows the temperature control means to be easily redistributed around the cover or the pouch after heating or cooling so that the user can ensure that there is an even distribution thereof to the eye and surrounding area (i.e. from above, below and either side of the eye socket), thereby maximising pain relief. Furthermore, it facilitates equalisation of the temperature throughout the mass of temperature control means, thereby reducing the risk of hotspots being created (when warmed), or ice "burn" occurring (when cooled), when the device is in use.

Preferably, the pouch is made of a material which effectively dissipates heat through the cover and towards a user's eye and surrounding area, when the device is in use. For example, the pouch may be made of a textile, such as cotton, silk, satin, or polyester. Cotton is preferred, especially in embodiments where the device is heated. However, in embodiments where the device is cooled, the pouch may comprise a waterproof material, for example latex or rubber. The pouch may comprise a label attached thereto, wherein the label comprises instructions for use of the device.

In one embodiment, the shape of the cover may be such that it fit's over only one eye of the subject, leaving the other eye exposed. Advantageously, this would allow the medical device to target a specific eye of a user.

In an alternative embodiment, the material and shape of the cover is such that it fits comfortably over both of the subject's eyes, and moulds therearound providing effective heat transfer. The cover preferably comprises a central section which is arranged, in use, to overlie the subject's nose, and two side sections disposed either side of the central section, which are arranged, in use, to overlie the subject's eyes and surrounding areas. Preferably, the maximum width of the central section is less than the maximum width of the side sections. For example, the width of the central section may be between about 6cm and 10cm across, or between about 7 and 9cm across. The width of each side section may be between about 10 and 15cm, or between about 10 and 13cm across. The maximum length of the cover is preferably at least 15cm, more preferably at least 18cm, and even more preferably at least 20cm.

The removable cover may comprise an opening through which the pouch may pass. The opening may be disposed on an external surface of the cover, preferably on the second side thereof. The second side is generally regarded as being the front surface of the device. The second side of the cover preferably comprises two pieces of material, which overlap each other thereby creating the opening. Preferably, the opening leads to a pocket disposed between the first and second sides of the cover, which pocket is configured to receive the pouch containing the temperature control means. The opening preferably extends diagonally across the first side of the cover, preferably along a line which extends across one of the side sections. The diagonal arrangement allows easier access of the pocket without interfering directly with the eye.

Preferably, the device comprises retaining means for securing the device (preferably the cover thereof) to the user's head and in position over the eye and/or surrounding area thereof. The retaining means may comprise at least one strap, which may be elasticated. Preferably, the retaining means comprises two straps, each strap being attached to mutually opposing ends of the cover, and arranged to be secured to each other to provide adjustability. Preferably, the retaining means comprises fastening means, for example one or more corresponding hook and loop fastening regions, such as Velcro regions.

According to a second aspect, there is provided a method of preparing the device of the first aspect for use in a method of treatment, the method comprising modulating the temperature of the device, and manipulating the device to redistribute the temperature control means around the cover.

According to a third aspect, there is provided a method of manufacturing a medical device, the method comprising:
- contacting at least a portion of a cover with a solution comprising an antimicrobial agent comprising silver, or a pharmacologically acceptable salt thereof, and an oxide, wherein the oxide is selected from the group consisting of copper oxide, iron oxide, zinc oxide, titanium dioxide and silica dioxide; and
- allowing the cover to dry;
wherein the cover is at least partially filled, or adapted to be at least partially filed, with temperature control means, and, in use, the cover is arranged to contact a subject's eye and/or a surrounding area thereof, and wherein the temperature control means is configured to modulate the temperature of the cover to thereby treat, prevent or ameliorate the eye condition.

Preferably, the method of the third aspect produces the medical device of the first aspect.

Allowing the cover to dry may comprise heating the cover above ambient temperature for a pre-determined time.

Accordingly, the method may comprise heating the cover to at least 30°C, preferably at least 40°C, more preferably at least 50°C, and most preferably at least 55°C. The method may comprise heating the cover to less than 150°C, preferably less than 100°C, more preferably less than 80°C, and most preferably less than 65°C. The method may comprise heating the cover to between 30°C and 150°C, preferably between 40°C and 100°C, more preferably between 50°C and 80°C, and most preferably between 55°C and 65°C.

The pre-determined time may be at least 5 minutes, preferably at least 30 or 60 minutes, more preferably at least 1 hour 30 minutes. The pre-determined time may be less than 5 hours, preferably less than 4 hours or 3 hours, more preferably less than 2 hours 30 minutes. The pre-determined time may be between 5 minutes and 5 hours, preferably between 30 minutes and 4 hours or between 60 minutes and 3 hours, more preferably between 1 hour 30 minutes and 2 hours 30 minutes.

The method may comprise allowing the cover to cool to room temperature.

According to a fourth aspect, there is provided a kit to make a medical device, the kit comprising
- a device comprising a cover which is at least partially filled with temperature control means; and
- a solution comprising an antimicrobial agent configured to be applied to the cover, wherein the antimicrobial agent comprises silver, or a pharmacologically acceptable salt thereof, and an oxide, wherein the oxide is selected from the group consisting of copper oxide, iron oxide, zinc oxide, titanium dioxide and silica dioxide;
wherein, in use, the cover is arranged to contact a subject's eye and/or a surrounding area thereof, and wherein the temperature control means is configured to modulate the temperature of the cover to thereby treat, prevent or ameliorate an eye condition.

Advantageously a person provided with the kit of the fourth aspect could conduct the method of the third aspect to create the medical device. Furthermore, the person could apply the solution after the device is washed, ensuring that the desired concentration of the antimicrobial agent is maintained.

All features described herein (including any accompanying claims, abstract and drawings), and/or all of the steps of any method or process so disclosed, may be combined with any of the above aspects in any combination, except combinations where at least some of such features and/or steps are mutually exclusive.

For a better understanding of the invention, and to show how embodiments of the same may be carried into effect, reference will now be made, by way of example, to the accompanying drawings, in which:-
**Figure 1** is a plan view from above of an outer cover of a medical device of the invention, which is used for treating eye conditions;
**Figure 2** is a plan view from below of the outer cover medical device shown in Figure 1;
**Figure 3** is a plan view of an inner pouch of the medical device;
**Figure 4** shows the complete medical device in use, when worn by a patient; and
**Figure 5** is a plan view from above of an outer cover of a further medical device of the invention, which is used for treating eye conditions.

### Examples

The inventors asked volunteers to wear prior art eye-masks. Testing of the eye-masks identified that bacteria was transferred to all of the eye masks tested, and in some instances mould was also transferred. The inventors have found that it is not possible to kill these microbes using a microwave.

The inventors work seeks to overcome this problem.

### Example 1 - The Medical Device

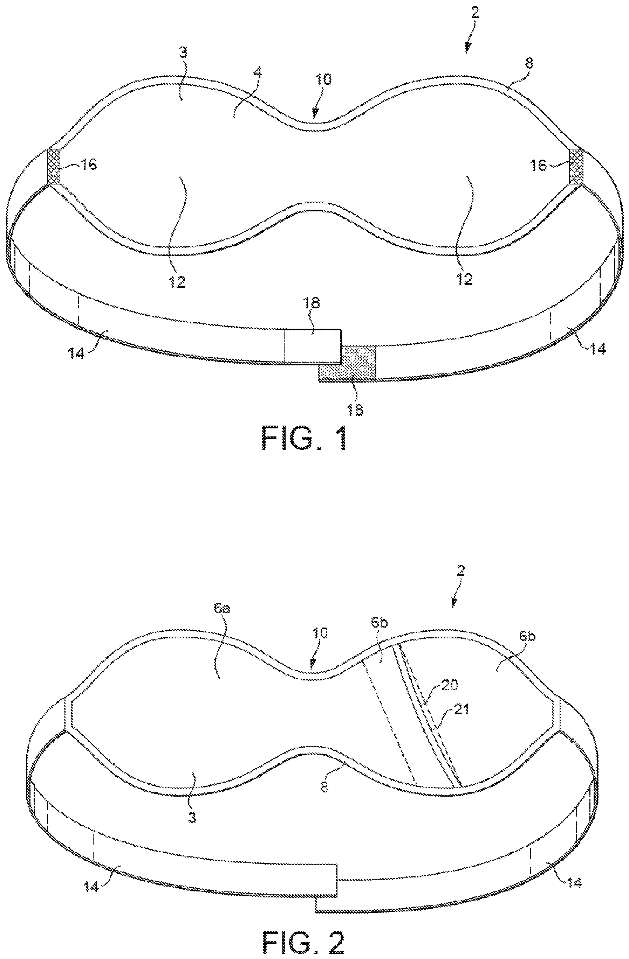

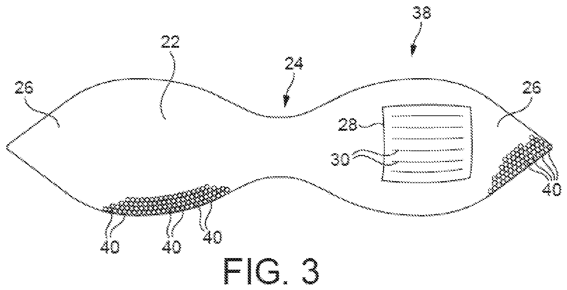

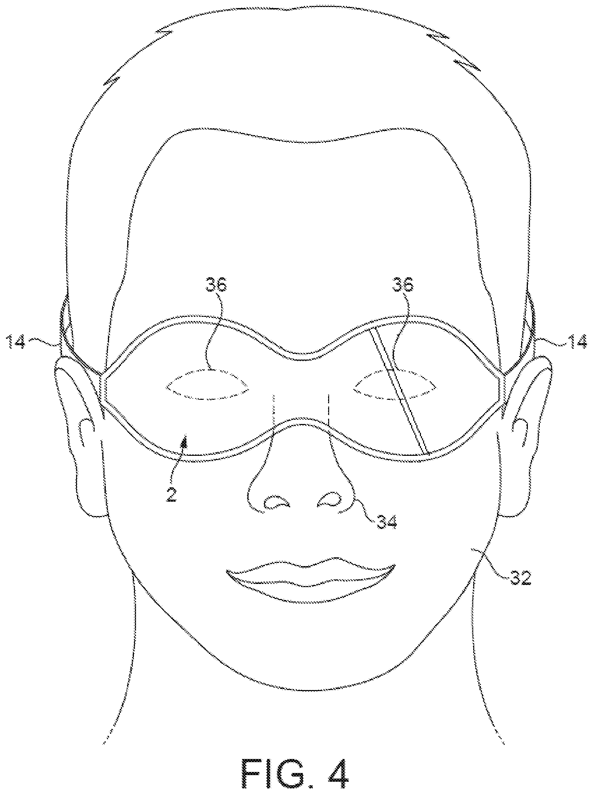

Referring to the Figures, there is shown a medical device 2, which consists of an outer cover 3 and an inner pouch 38 disposed therein. Figures 1 and 2 illustrate various views of the cover 3, Figure 3 shows the pouch 38, and Figure 4 shows the device 2 in position on a subject 32. In use, the device 2 is either heated by a conventional microwave, or cooled in a conventional fridge/freezer, and is effectively used to treat or even prevent a very wide range of eye conditions, such as styes and cysts, inflammation, dry eye disease, grittiness and irritation, itchy eyes (e.g. caused by hay fever), Meibomian Gland Dysfunction (MGD), chalazion, blepharitis and conjunctivitis, and so on, depending on whether heating or cooling of the eyes and the associated areas is desired. It will be appreciated however that this list of eye conditions is not exhaustive and that the device 2 can be used to treat or prevent any kind of eye pain or condition, or to soothe a headache, which may have been caused by eye strain. Use of the medical device 2 is the first step in eye/eyelid hygiene followed by the application of eyedrops etc to treat an eye condition. Effective use of the device 2 improves eye hygiene such that the chances of many eye conditions occurring are significantly reduced or prevented.

Referring first to Figure 1, the back of the outer cover 3 of the device 2 is shown, and is composed of a single piece of material 4, for example soft cotton or silk. The rear piece of material 4 is generally light blue or white in colour, and so will not emit heat to the same extent as a black piece of material would. As described in more detail in example 3, below, the rear piece of material also comprises an antimicrobial agent.

Referring to Figure 2, the front of the outer cover 3 of the device 2 is shown, and is composed of two pieces of material 6a, 6b, for example soft cotton or silk. In the embodiment illustrated, the two pieces of material 6a, 6b comprise a phase change material (PCM) which is configured to absorb, store and release heat. Suitable PCMs are sold under the trade mark Outlast^{®} and are described in a variety of patent applications, for instance see EP2558504, EP2501846, EP2264231 and EP2392712. The PCM helps to thermally regulate the temperature of the device 2, ensuring a roughly consistent temperature is delivered through the rear piece of material 4 for an extended period of time.

The perimeter of the front piece of material 4 is stitched to the two rear pieces of material 6a, 6b by a line of stitching 8, and rear pieces 6a, 6b are stitched to each other only at their peripheral edges, as shown in Figure 2.

As can be seen in Figures 1 and 2, an adjustable comfort strap 14 is secured to each side of the cover 3. Each strap 14 is approximately 20cm in length, and is stitched 16 to each side of the rear piece of material 4. As can be seen in Figure 1, each end of the straps 14 distal from the stitching 16 is covered with an area of Velcro^{®} 18. In use, the subject 18 can easily adjust the tightness of the outer cover 3 around the head and over the eyes 36 by varying the combined length of the straps 14 via the respective positioning of the Velcro (RTM) regions 18.

As shown in Figure 4, the shape of the cover 3 is such that it fits comfortably around the subject's head and over their eyes 36. The cover 3 has a narrow central section 10 which is arranged, in use, to overlie the subject's nose 34, and two wide side sections 12 disposed either side of the central section 10, which are arranged, in use, to overlie the subject's eyes 36. The width of the central section 10 is about 8cm across and is therefore narrower than the width of each side section 12, which is about 10cm across. The length of the entire cover 3 (excluding the straps 14) is approximately 25cm across.

As shown in Figure 2, the front piece of material 6a forming the outer cover 3 extends across about 75% of the width of the rear of the cover 3, and over a small section of the front piece of material 6b, thereby creating a small overlap. The pieces 6a, 6b are not stitched together at the overlap, and so an opening 20 is created, which leads to a pocket 21 that is disposed between the rear piece of material 4 and the two front pieces of material 6a, 6b. The opening 20 extends diagonally across the front surface 6 of the cover 3, generally along a side region 12 thereof. The opening 20 is suitably sized to enable the insertion of the inner pouch 38, which is shown in detail in Figure 3, and described below.

Referring now to Figure 3, the inner pouch 38 consists of two pieces of thin material, such as cotton, which are stitched together to create a flexible sack or bag. The material of the pouch 38 may also comprise an antimicrobial agent. The shape of the pouch 38 resembles that of the front 4 and rear 6 surfaces of the outer cover 3, although the dimensions are slightly less so that the pouch 38 can be easily removed and inserted though the opening 20 in the cover 3. Thus, as can be seen in the Figure, the pouch 38 has a narrow central region 24, which corresponds to the narrow central region 10 of the outer cover 3, and wider side regions 26, which correspond to the wider side regions 12 of the cover 3. Stitched to one of the side regions 26 of the pouch 38, there is provided a label 28 on which instructions 30 for use are clearly printed.

In a first embodiment of the device 2, the pouch 38 contains a plurality of microwaveable grains or seeds 40. For instance, the grains may be a special mix of natural seeds (e.g. 33.3% canary seed, 33.3% rapeseed, and 33.3% millet seed), which has been developed to ensure that the device 2 warms to the correct temperatures in the least amount of time, and then retains the heat for as long as possible, and at a comfortable level when worn by the subject 32. Other grains that may be used are flax seed or wheat, either alone or in combination with the other grains described herein. Alternatively, silica beads can also be used, and are described in more detail in WO 2015/011455 A1. This embodiment is used for microwaving and transferring heat to the subject 32, as will be described below.

The embodiment of the device 2 described above may also be used to apply a cooling effect to a subject's eyes. Alternatively, in a second embodiment of the device 2, where the subject's eyes 36 require a cooling effect, instead of being filled with microwaveable grains, seeds and/or beads 40, the pouch 38 contains a cooling gel or alternatively polymer cooling crystals, or the like (not shown). An example of a suitable cooling gel is available from Yangzhou Tianle Hot Water Bottle Factory (China). In use, when exposed to cold temperatures (i.e. from a conventional fridge or freezer), the gel cools down and retains the cool temperature. Suitable cooling crystals (available from N-rit co., ltd, #223-427, Seoknam2-Dong, Seo-gu, Incheon City, Korea 404-825), can also be used to cool down the device 2, but without the need for a freezer. To cool the device 2, it is first immersed in cold water for 3-5 minutes until the high-tech polymer crystals fully absorb the water and become "activated". It may be desirable to also place the activated crystals in a fridge for 30 minutes for an even greater cooling effect. The crystals are made of a non-toxic crystal polymer, which becomes cold, and has a long-lasting cooling effect. The subject 32 can enjoy the soothing, cooling effect imparted by the crystals.

For clarity, Figure 3 does not show a full complement of grains, seeds and/or beads 40 (or cooling gel/crystals); instead it shows, for illustrative purposes only, small groupings of grains 40 at various positions within the pouch 38. However, it should be appreciated that the pouch 38 contains enough grains, seeds and/or beads 40 (or cooling gel/crystals) to be fully dispersed throughout, such that the subject's eyes 36 and surrounding areas are fully covered when the device 2 is placed in the correct position. Conversely, the pouch 38 is not completely filled with grains, seeds and/or beads 40 (or cooling gel/crystals) so that the subject 32 can easily move them around the inside the pouch 38 to avoid hot/cold spots. Also, the subject 32 is able to easily insert the pouch 38 through opening 20 into the pocket area 21. As a guide, the pouch 38 is filled to about 50-70% of its capacity with the grains, seeds and/or beads 40 (or cooling gel/crystals).

While not illustrated, it will be appreciated that in an alternative embodiment the grains, seeds and/or beads 40 (or cooling gel/crystals) may be disposed directly in the cover 3. Accordingly, in this embodiment, the device 2 would not comprise a pouch 38. Furthermore, the front of the cover 3 may comprise a single piece of fabric, such that the cover does not comprise an opening 20.

In use, the subject 32 must first remove the inner pouch 38 from the outer cover 3 by carefully pulling it out. The relatively sizes of the pouch 38 and cover 3 mean that it is not a very tight fit, and so there is no risk that the pouch would be ripped and lose its contents during the removal operation. The subject 32 then places the pouch 38 on a clean microwaveable plate, and heats it on full power (based on a 850-1000 Watt microwave) for approximately 30 seconds. Heating times should never be exceeded. The microwave energy is absorbed by the grains 40, which are therefore heated up to an elevated temperature of about 42-47°C. After heating, the pouch 38 is then inserted into the outer cover 3 through the opening 20 and then arranged such that it fits well within the pocket area 21, in an extended configuration therein, i.e. the positions of the central region 24 and side regions 26 of the pouch 38 correspond with the positions of the central region 10 and side regions 12 of the outer cover 3.

Alternatively, in the embodiment where the grains, seeds and/or beads 40 (or cooling gel/crystals) are disposed directly in the cover 3, the subject 32 may simply heat the cover 3 in the microwaveable.

In order to distribute the heat effectively, and to prevent any "hot spots" developing, the device 2 can be shaken during (e.g. about half-way) through the heating period, and again after heating, but before use. Once fully heated, the subject 32 then carefully checks the temperature of the grains 40 by touching the outer cover 3 (now containing the heated pouch 38) on to the inside of the wrist, before putting on the eyes 36. To avoid damage to the eyeball, the subject 32 must close the eyes 36 prior to gently resting the outer cover 3 of the device 2 in place, as illustrated in Figure 4. The rear 4 of the outer cover 3 is contacted on the subject's eyes, and the front 6 is exposed to the air.

The straps 14 are then fastened together via the Velcro (RTM) 18 forming a headband. The pressure that is applied by the device 2 to the subject's eyes 36 can be adjusted by tightening or loosening the straps 14 to suit personal comfort. The device 2 gently conforms and moulds to the contours of the eyes 36 and shape of the subject's face, and is left in position for 7 to 10 minutes, or longer if desired. Due to the two pieces of material 6a, 6b comprising the PCM, the inventors have found that the device is capable of maintaining the temperature between 42°C and 47°C in excess of ten minutes. The straps 14 provide tight support and even distribution of the grain 40 to the eyes and surrounding areas, thereby maximising pain relief.

After using the device 2, with the eyes closed, the subject 32 then gently massages the upper eyelid by smoothing a finger from the inner corner of the eye 36 to the outer corner just above the lashes. This procedure is then repeated just below the lashes on the lower lid. Debris should then be removed from the subject's eyes 36 and the surrounding area. The exercise should be repeated for about a minute on both eyes. The device can be used two or three times a day, but can be reduce to 2-3 times a week once the symptoms improve.

It is widely recognised and accepted that warmth eases pain and helps movement in muscles and joints, by increasing vasodilation and therefore blood flow to restricted areas. Accordingly, the device 2 is useful for treating a range of conditions, which would benefit from such soothing heat treatment. However, it is also possible to use the device 2 when it is cold, rather than warm (following microwaving). Applying the device 2 when cold reduces pain and swelling by causing blood vessels to vasoconstrict. It has also been demonstrated by hay fever sufferers that cooling the eyes 36, especially the corners thereof, results in a satisfying reduction in itchiness and irritation.

When wishing to cool the eyes 36, the pouch 38 is first removed from the outer cover, and then placed inside a receptacle, such as a clean plastic bag (not shown), and left in the fridge or freezer for at least an hour. The cooled pouch 38 is then inserted back into the outer cover 3, which is then gently placed over the eyes 36 and nose 34. The subject 32 then increases the pressure of the device 2 over the eyes via the straps 14, as described above, and allows the cool soothing effect to act. It is highly unlikely that the user would get ice burns from using the device 2 when chilled. However, for safety, it is preferred that the device 2 is not used for more than 10 minutes at any one time.

### Example 2 - Antimicrobial Properties

An antimicrobial solution comprising a biguanide polymer with a molecular weight between 13,000 and 23,000 as an antimicrobial agent was produced.

A trigger spray was used to apply the antimicrobial solution first to the internal surface of the back of the outer cover 3, then to the external surface of the outer cover 3. The devices 2 were then dried in a convection oven at 60°C for 2 hours and were then allowed to cool to room temperature for 2 hours.

A solution comprising test species was prepared. The solution comprised:

| | |
|---|---|
| *Pseudomonas aeruginosa* | 8.3 x 10⁶ CFU/g |
| *Staphylococcus aureus* | 2.8 x 10⁶ CFU/g |
| *Candida albicans* | 3.8 x 10⁶ CFU/g |
| *Aspergillus brasiliensis* | 6.0 x 10⁵ CFU/g |

The solution comprising the test species was then spread over the external surface of the outer cover 3. After 10 minutes the external surface of the outer cover 3 was swabbed to measure the recovery of live micro-organisms. The results are given in Table 1, below. The mass of active agent was calculated assuming that 30% of the added weight is the active agent. The concentration of the active agent was calculated assuming that the average molecular weight of the active agent was 18,000 and the surface area of the blind fold was 500 cm² (250 cm² for the internal surface and 250 cm² for the external surface).

**Table 1: Results of application of antimicrobial solution to the outer cover 3 of the device 2**

| **Sample** | **Initial mass / g** | **Mass after 1st spray / g** | **Mass after 2nd spray / g** | **Mass after drying for 2 hours at 65°C / g** | |
|---|---|---|---|---|---|
| 1 | 13.71 | 19.13 | 21.59 | 13.59 | |
| 2 | 13.75 | 19.14 | 21.63 | 13.67 | |
| 3 | 13.79 | 18.84 | 21.77 | 13.71 | |
| 4 | 13.85 | 18.31 | 22.11 | 13.57 | |
| 5 | 13.76 | 19.21 | 21.59 | 13.65 | |
| 6 | 13.69 | 18.31 | 21.63 | 13.62 | |
| 7 | 14.02 | 19.77 | 21.77 | 13.97 | |
| 8 | 13.82 | 19.11 | 22.11 | 13.67 | |

| **Sample** | **Mass after cooling to room temperature / g** | **Added mass / g** | **Mass of active agent added to cover / g** | **Concentration of active agent on cover / mol/cm²** | **Number of colony forming units/cm² detected on sample** |
|---|---|---|---|---|---|
| 1 | 13.8 | 0.09 | 0.027 | 3.00E-09 | 0 |
| 2 | 13.84 | 0.09 | 0.027 | 3.00E-09 | 0 |
| 3 | 13.89 | 0.1 | 0.03 | 3.33E-09 | 0 |
| 4 | 13.95 | 0.1 | 0.03 | 3.33E-09 | 0 |
| 5 | 13.88 | 0.12 | 0.036 | 4.00E-09 | 0 |
| 6 | 13.79 | 0.1 | 0.03 | 3.33E-09 | 0 |
| 7 | 14.14 | 0.12 | 0.036 | 4.00E-09 | 0 |
| 8 | 13.95 | 0.13 | 0.039 | 4.33E-09 | 0 |

It will be noted that the antimicrobial solution was effectively able to kill all of the test species.

### Example 3 - Toxicity Tests

An agar overlay test was designed to determine the cytotoxicity of the antimicrobial solution when applied to the outer cover 3.

Six well cell culture plates were seeded with a verified quantity of industry standard L-929 cells (ATCC CCL-1) and incubated at 37 ± 1°C with 5 ± 1% CO2 until approximately 80% confluent. The agar overlay consisted of an equal mixture of 1% noble agar and 2X Minimal Essential Media + 10% bovine calf serum. Solid test articles were placed directly on the solidified agar overlay testing ≥ 100 mm² per test well. Positive and negative reference controls were included with each assay.

All tests were performed using three test wells per test article. After the addition of the test articles, the cell culture plates were incubated as described above for 24-26 hours. Following incubation, cells were evaluated microscopically using the evaluation criteria outline in table 2.

**Table 2: Evaluation criteria to score cells**

| **Grade** | **Description of Zone** |
|---|---|
| 0 | No detectable zone around or under the test article |
| 1 | Some malformed or degenerate cells under the test article |
| 2 | Zone limited to area under the test article and less than 0.45 cm beyond the test article |
| 3 | Zone extends 0.45 to 1.0 cm beyond the test article. |
| 4 | Zone extends greater than 1 cm, beyond the test article |

An antimicrobial solution comprising silver chloride and titanium dioxide, in a weight ratio of 1:4, as an antimicrobial agents was produced and applied to a cotton fabric. Similar, treated fabric may be obtained from Polygiene^{®}. The cotton fabric was used as the test sample. Polypropylene pellets were used as a negative control and latex natural rubber was used as a positive control.

The results from the agar overlay test are given in table 3.

**Table 3: Results of agar overlay test**

| **Article** | **Scores** | | | |
|---|---|---|---|---|
| | **#1** | **#2** | **#3** | **Average** |
| Cotton treated with antimicrobial solution | 1 | 1 | 1 | 1 |
| Polypropylene pellets | 0 | 0 | 0 | 0 |
| Latex natural rubber | 4 | 4 | 4 | 4 |

The United States Pharmacopeia & National Formulary (USP <87>) states that the test article meets the requirements if the reactivity grade is not greater than grade 2 or a mild reactivity. The ANSI/AAMI/ISO 10993-5 standard states that the achievement of a numerical grade greater than 2 is considered a cytotoxic effect.

As indicated above, the cotton treated with antimicrobial solution received a score of 1, and so is not deemed to be cytotoxic.

### Example 4 - Further Antimicrobial Tests

As explained in Example 3, an antimicrobial solution comprising silver chloride and titanium dioxide, in a weight ratio of 1:4, as an antimicrobial agents was produced and applied to a cotton fabric. A sample of the treated cotton fabric was washed equivalently to fifteen domestic cycles at 40°C to determine if the treated fabric maintained its antimicrobial properties after washing.

An inoculum of *Staphylococcus aureus* prepared in 1:500 Nutrient Broth was prepared, and 0.2 ml samples of the inoculum were applied to replicate (3) samples of either the untreated fabric, the treated fabric or the treated and washed fabric. The number of colony forming units/cm² was calculated at the time of inoculation and then 24 hours later. The results are shown below in Table 4.

**Table 4: Antimicrobial activity of treated fabric**

| **Sample** | **Number of colony forming units/cm² on sample** | |
|---|---|---|
| | **0hrs** | **24hrs** |
| Treated fabric | 9300 | < 6.25 |
| Treated and washed fabric | 9300 | < 6.25 |
| Untreated fabric | 9300 | 3600 |

The results show that the treated fabric has an antimicrobial effect reducing the bacteria to below the limit of detection over 24 hours. This equates to a >3.17 log kill of the bacteria over the 24 hours. The same effect was observed for the treated fabric which had been washed fifteen times, showing that the fabric retains its antimicrobial properties after washing.

Conversely, the untreated fabric only showed a 0.41 log kill of the bacteria over 24 hours.

### Example 5 - A Further Medical Device

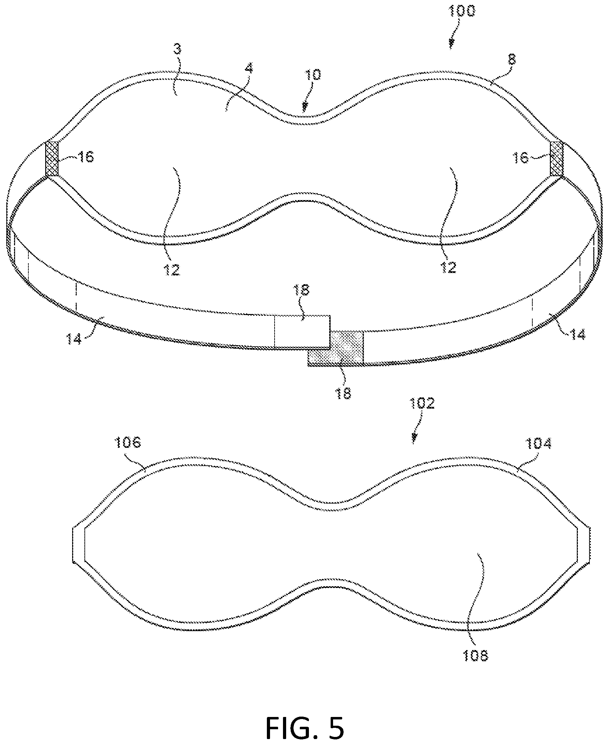

A further medical device 100 is shown in Figure 5. Like the medical device 2 discussed in Example 1, the medical device 100 consists of an outer cover 3 and an inner pouch 38 disposed therein. However, unlike the device 2 discussed in example 1, the outer cover 3 of the device 2 does not comprise an antimicrobial agent.

Instead, a disposable barrier 102 is provided to place over the back of the outer cover 3 before use. The disposable barrier 102 is sized and shaped to substantially correspond to the back of the outer cover 3. The disposable barrier 102 comprises a sterile cotton gauze 104 with an adhesive disposed on a first side 106 thereof, and a backing strip 107 disposed over the adhesive. The disposable barrier 102 is disposed in a sterile pouch (not shown) prior to use. Accordingly, when a user wishes to use the device 2, they may heat it as described in example 1. They can then remove the disposable barrier 102 from the pouch, remove the backing strip 108 and affix the disposable barrier 102 to the back of the outer cover 3 before placing the device against their eyes 36.

After use, the user can remove the disposable cover 102 from the outer cover 3 and dispose of it. Accordingly, the disposable cover 102 prevents microbes being transferred between the patient's eyes 36 and the outer cover 3.

### Conclusions

Advantages of the device 2 reside in the antimicrobial agent being able to effectively kill bacteria and fungus, thereby preventing infection or reinfection of a subject's eyes. Furthermore, the PCM material thermally regulate the temperature of the device 2, ensuring a roughly consistent temperature is delivered to the eyes of the subject for an extended period of time.

Furthermore, the device 100 also prevents transfer of microbes between the patient's eyes 36 and the outer cover 3, also preventing infection or reinfection of a subject's eyes.

## Claims

1. A medical device (2) for treating, preventing or ameliorating an eye condition, the device (2) comprising a cover (3), at least a portion of which comprises an antimicrobial agent and is at least partially filled with temperature control means (40), wherein, in use, the cover (3) is arranged to contact a subject's eye and/or a surrounding area thereof, and wherein the temperature control means (40) is configured to modulate the temperature of the cover (3) to thereby treat, prevent or ameliorate the eye condition **characterised in that**:
the antimicrobial agent comprises silver, or a pharmacologically acceptable salt thereof, and an oxide, wherein the oxide is selected from the group consisting of copper oxide, iron oxide, zinc oxide, titanium dioxide and silica dioxide.

2. A medical device (2) according to claim 1, wherein the antimicrobial agent comprises a silver salt, optionally wherein the silver salt is selected from the group consisting of silver sulphate, silver nitrate, silver chloride, silver sodium zirconium hydrogen phosphate and silver sulfadiazine.

3. A medical device (2) according to any preceding claim, wherein the oxide is titanium dioxide.

4. A medical device (2) according to any preceding claim, wherein the weight ratio of the silver, or the salt thereof, to the oxide is between 5:1 and 1:20.

5. A medical device (2) according to any preceding claim, wherein an external surface of the first side of the cover (3) comprises the antimicrobial agent.

6. A medical device (2) according to any preceding claim, wherein an internal surface of the first side (3) of the cover comprises the antimicrobial agent.

7. A medical device (2) according to any preceding claim, wherein the concentration of the antimicrobial agent is at least 1 x 10⁻¹¹ mol/cm², or at least 1 x 10⁻¹⁰ mol/cm², or at least 5 x 10⁻¹⁰ mol/cm², or at least 1 x 10⁻⁹ mol/cm², or at least 2 x 10⁻⁹ mol/cm², or at least 3 x 10⁻⁹ mol/cm².

8. A medical device (2) according to any preceding claim, wherein a first side of the cover (3) is made of a first material, which dissipates heat when the device is in use, at a greater rate than that of a second side of the cover (3) made of a second material comprising a phase change material (PCM).

9. A medical device (2) according to claim 8, wherein the second material comprises a fabric with a layer of the PCM disposed thereon, preferably wherein the layer of the PCM is disposed on an internal surface of the fabric.

10. A medical device (2) according to either claim 8 or claim 9, wherein the PCM has a transition temperature between 30 and 70°C, or between 40 and 60°C, or between 42 and 47°C.

11. A medical device (2) according to any preceding claim, wherein the device (2) comprises a pouch (38) disposed within the cover (3) and at least partially filled with the temperature control means (40), wherein the cover (3) is removable such that the pouch (38) is removably disposed therein, and/or wherein the temperature control (40) means comprises grain, seed, tourmaline beads, silica beads or silica gel.

12. A method of preparing the device (2) according to any preceding claim for use in a method of treatment, the method comprising modulating the temperature of the device (2), and manipulating the device (2) to redistribute the temperature control means around the cover.

13. A method of manufacturing a medical device (2), the method comprising:
- contacting at least a portion of a cover (3) with a solution comprising an antimicrobial agent comprising silver, or a pharmacologically acceptable salt thereof, and an oxide, wherein the oxide is selected from the group consisting of copper oxide, iron oxide, zinc oxide, titanium dioxide and silica dioxide; and
- allowing the cover (3) to dry;
wherein the cover (3) is at least partially filled, or adapted to be at least partially filed, with temperature control means (40), and, in use, the cover (3) is arranged to contact a subject's eye and/or a surrounding area thereof, and wherein the temperature control means (40) is configured to modulate the temperature of the cover (3) to thereby treat, prevent or ameliorate the eye condition.

14. A method according to claim 13, wherein allowing the cover (3) to dry comprises heating the cover (3) to between 30°C and 150°C, or between 40°C and 100°C, or between 50°C and 80°C, or between 55°C and 65°C for a pre-determined time, optionally wherein the pre-determined time is at least 5 minutes, or at least 30 or 60 minutes, or at least 1 hour 30 minutes.

15. A kit to make a medical device (2), the kit comprising
- a device comprising a cover (3) which is at least partially filled with temperature control means (40); and
- a solution comprising an antimicrobial agent configured to be applied to the cover, wherein the antimicrobial agent comprises silver, or a pharmacologically acceptable salt thereof, and an oxide, wherein the oxide is selected from the group consisting of copper oxide, iron oxide, zinc oxide, titanium dioxide and silica dioxide;
wherein, in use, the cover (3) is arranged to contact a subject's eye and/or a surrounding area thereof, and wherein the temperature control means (40) is configured to modulate the temperature of the cover (3) to thereby treat, prevent or ameliorate an eye condition.

## Patentansprüche

1. Medizinische Vorrichtung (2) zum Behandeln, Verhindern oder Verbessern eines Augenzustands, wobei die Vorrichtung (2) eine Abdeckung (3) umfasst, von der zumindest ein Teil ein antimikrobielles Mittel umfasst und die zumindest teilweise mit Temperatursteuermittel (40) gefüllt ist, wobei in Verwendung die Abdeckung (3) angeordnet ist, um das Auge eines Subjekts und/oder einen umgebenden Bereich davon zu kontaktieren, und wobei das Temperatursteuermittel (40) konfiguriert ist, um die Temperatur der Abdeckung (3) zu modulieren, um dadurch den Augenzustand zu behandeln, zu verhindern oder zu verbessern, **dadurch gekennzeichnet, dass**: das antimikrobielle Mittel Silber oder ein pharmakologisch annehmbares Salz davon und ein Oxid umfasst, wobei das Oxid ausgewählt ist aus der Gruppe bestehend aus Kupferoxid, Eisenoxid, Zinkoxid, Titandioxid und Siliziumdioxid.

2. Medizinische Vorrichtung (2) nach Anspruch 1, wobei das antimikrobielle Mittel ein Silbersalz umfasst, wobei optional das Silbersalz ausgewählt ist aus der Gruppe bestehend aus Silbersulfat, Silbernitrat, Silberchlorid, Silbernatriumzirkoniumhydrogenphosphat und Silbersulfadiazin.

3. Medizinische Vorrichtung (2) nach einem vorhergehenden Anspruch, wobei das Oxid Titandioxid ist.

4. Medizinische Vorrichtung (2) nach einem vorhergehenden Anspruch, wobei das Gewichtsverhältnis des Silbers oder des Salzes davon zu dem Oxid zwischen 5:1 und 1:20 ist.

5. Medizinische Vorrichtung (2) nach einem vorhergehenden Anspruch, wobei eine Außenfläche der ersten Seite der Abdeckung (3) das antimikrobielle Mittel umfasst.

6. Medizinische Vorrichtung (2) nach einem vorhergehenden Anspruch, wobei eine Innenfläche der ersten Seite (3) der Abdeckung das antimikrobielle Mittel umfasst.

7. Medizinische Vorrichtung (2) nach einem vorhergehenden Anspruch, wobei die Konzentration des antimikrobiellen Mittels zumindest 1 x 10⁻¹¹ mol/cm² oder zumindest 1 x 10⁻¹⁰ mol/cm² oder zumindest 5 x 10⁻¹⁰ mol/cm² oder zumindest 1 x 10⁻⁹ mol/cm² oder zumindest 2 x 10⁻⁹ mol/cm² oder zumindest 3 x 10⁻⁹ mol/cm² ist.

8. Medizinische Vorrichtung (2) nach einem vorhergehenden Anspruch, wobei eine erste Seite der Abdeckung (3) aus einem ersten Material gefertigt ist, das Wärme, wenn die Vorrichtung in Verwendung ist, mit einer größeren Rate als diejenige einer zweiten Seite der Abdeckung (3), die aus einem zweiten Material gefertigt ist, das ein Phasenwechselmaterial (PCM) umfasst, ableitet.

9. Medizinische Vorrichtung (2) nach Anspruch 8, wobei das zweite Material ein Gewebe mit einer darauf vorgesehenen Schicht des PCM umfasst, wobei bevorzugt die Schicht des PCM auf einer Innenfläche des Gewebes vorgesehen ist.

10. Medizinische Vorrichtung (2) nach Anspruch 8 oder Anspruch 9, wobei das PCM eine Übergangstemperatur zwischen 30 und 70 °C oder zwischen 40 und 60 °C oder zwischen 42 und 47 °C aufweist.

11. Medizinische Vorrichtung (2) nach einem vorhergehenden Anspruch, wobei die Vorrichtung (2) einen Beutel (38) umfasst, der innerhalb der Abdeckung (3) vorgesehen und zumindest teilweise mit dem Temperatursteuermittel (40) gefüllt ist, wobei die Abdeckung (3) entfernbar ist, sodass der Beutel (38) entfernbar darin vorgesehen ist, und/oder wobei das Mittel zur Temperatursteuerung (40) Korn, Samen, Turmalinkügelchen, Siliziumkügelchen oder Siliziumgel umfasst.

12. Verfahren zum Herstellen der Vorrichtung (2) nach einem vorhergehenden Anspruch zur Verwendung in einem Behandlungsverfahren, wobei das Verfahren Modulieren der Temperatur der Vorrichtung (2) und Manipulieren der Vorrichtung (2) umfasst, um das Temperatursteuermittel um die Abdeckung neu zu verteilen.

13. Verfahren zum Herstellen einer medizinischen Vorrichtung (2), wobei das Verfahren Folgendes umfasst:
- Kontaktieren von zumindest einem Teil einer Abdeckung (3) mit einer Lösung, die ein antimikrobielles Mittel umfasst, das Silber oder ein pharmakologisch annehmbares Salz davon und ein Oxid umfasst, wobei das Oxid ausgewählt ist aus der Gruppe bestehend aus Kupferoxid, Eisenoxid, Zinkoxid, Titandioxid und Siliziumdioxid; und
- Ermöglichen, dass die Abdeckung (3) trocknet;
wobei die Abdeckung (3) zumindest teilweise mit Temperatursteuermittel (40) gefüllt oder ausgelegt ist, um zumindest teilweise damit gefüllt zu sein, und in Verwendung die Abdeckung (3) angeordnet ist, um das Auge eines Subjekts und/oder einen umgebenden Bereich davon zu kontaktieren, und wobei das Temperatursteuermittel (40) konfiguriert ist, um die Temperatur der Abdeckung (3) zu modulieren, um dadurch den Augenzustand zu behandeln, zu verhindern oder zu verbessern.

14. Verfahren nach Anspruch 13, wobei das Ermöglichen, dass die Abdeckung (3) trocknet, Erhitzen der Abdeckung (3) auf zwischen 30 °C und 150 °C oder zwischen 40 °C und 100 °C oder zwischen 50 °C und 80 °C oder zwischen 55 °C und 65 °C für eine vorbestimmte Zeit umfasst, wobei optional die vorbestimmte Zeit zumindest 5 Minuten oder zumindest 30 oder 60 Minuten oder zumindest 1 Stunde 30 Minuten ist.

15. Kit zum Fertigen einer medizinischen Vorrichtung (2), wobei das Kit Folgendes umfasst:
- eine Vorrichtung, die eine Abdeckung (3) umfasst, die zumindest teilweise mit Temperatursteuermittel (40) gefüllt ist; und
- eine Lösung, die ein antimikrobielles Mittel umfasst, die konfiguriert ist, um auf die Abdeckung aufgebracht zu werden, wobei das antimikrobielle Mittel Silber oder ein pharmakologisch annehmbares Salz davon und ein Oxid umfasst, wobei das Oxid ausgewählt ist aus der Gruppe bestehend aus Kupferoxid, Eisenoxid, Zinkoxid, Titandioxid und Siliziumdioxid;
wobei in Verwendung die Abdeckung (3) angeordnet ist, um das Auge eines Subjekts und/oder einen umgebenden Bereich davon zu kontaktieren und wobei das Temperatursteuermittel (40) konfiguriert ist, um die Temperatur der Abdeckung (3) zu modulieren, um dadurch einen Augenzustand zu behandeln, zu verhindern oder zu verbessern.

## Revendications

1. Dispositif médical (2) destiné à traiter, prévenir ou améliorer un état oculaire, le dispositif (2) comprenant un couvercle (3), dont au moins une partie comprend un agent antimicrobien et est au moins partiellement remplie d'un moyen de régulation de température (40), lors de l'utilisation, ledit couvercle (3) étant agencé de manière à entrer en contact avec l'œil d'un sujet et/ou une zone environnante de celui-ci, et ledit moyen de régulation de température (40) étant conçu pour moduler la température du couvercle (3) de manière à ainsi traiter, prévenir ou améliorer l'état oculaire, **caractérisé en ce que** : l'agent antimicrobien comprend de l'argent, ou un sel pharmacologiquement acceptable de celui-ci, et un oxyde, ledit oxyde étant choisi dans le groupe constitué par l'oxyde de cuivre, l'oxyde de fer, l'oxyde de zinc, le dioxyde de titane et le dioxyde de silice.

2. Dispositif médical (2) selon la revendication 1, ledit agent antimicrobien comprenant un sel d'argent, éventuellement ledit sel d'argent étant choisi dans le groupe constitué par le sulfate d'argent, le nitrate d'argent, le chlorure d'argent, l'hydrogénophosphate de zirconium, de sodium et d'argent et la sulfadiazine d'argent.

3. Dispositif médical (2) selon l'une quelconque des revendications précédentes, ledit oxyde étant le dioxyde de titane.

4. Dispositif médical (2) selon l'une quelconque des revendications précédentes, ledit rapport pondéral de l'argent, ou de son sel, à l'oxyde étant situé entre 5:1 et 1:20.

5. Dispositif médical (2) selon l'une quelconque des revendications précédentes, une surface externe du premier côté du couvercle (3) comprenant l'agent antimicrobien.

6. Dispositif médical (2) selon l'une quelconque des revendications précédentes, une surface interne du premier côté (3) du couvercle comprenant l'agent antimicrobien.

7. Dispositif médical (2) selon l'une quelconque des revendications précédentes, ladite concentration de l'agent antimicrobien étant d'au moins 1 x 10⁻¹¹ mol/cm², ou d'au moins 1 x 10^{7 10} mol/cm², ou d'au moins 5 x 10⁻¹⁰ mol/cm², ou d'au moins 1 x 10⁻⁹ mol/cm², ou d'au moins 2 x 10⁻⁹ mol/cm², ou d'au moins 3 x 10⁻⁹ mol/cm².

8. Dispositif médical (2) selon l'une quelconque des revendications précédentes, un premier côté du couvercle (3) étant constitué d'un premier matériau, qui dissipe la chaleur lorsque le dispositif est en cours d'utilisation, à une vitesse supérieure à celle d'un second côté du couvercle (3) constitué d'un second matériau comprenant un matériau à changement de phase (PCM).

9. Dispositif médical (2) selon la revendication 8, ledit second matériau comprenant un tissu sur lequel est disposée une couche du PCM, ladite couche du PCM étant de préférence disposée sur une surface interne du tissu.

10. Dispositif médical (2) selon la revendication 8 ou la revendication 9, ledit PCM présentant une température de transition située entre 30 et 70°C, ou entre 40 et 60°C, ou entre 42 et 47°C.

11. Dispositif médical (2) selon l'une quelconque des revendications précédentes, ledit dispositif (2) comprenant une poche (38) disposée à l'intérieur du couvercle (3) et au moins partiellement remplie du moyen de régulation de température (40), ledit couvercle (3) étant amovible de sorte que la poche (38) soit disposée de manière amovible en son sein, et/ou ledit moyen de régulation (40) de température comprenant des grains, des graines, des billes de tourmaline, des billes de silice ou du gel de silice.

12. Procédé de préparation du dispositif (2) selon l'une quelconque des revendications précédentes destiné à être utilisé dans un procédé de traitement, le procédé comprenant la modulation de la température du dispositif (2), et la manipulation du dispositif (2) de manière à redistribuer le moyen de régulation de température autour du couvercle.

13. Procédé de fabrication d'un dispositif médical (2), le procédé comprenant :
- la mise en contact d'au moins une partie d'un couvercle (3) avec une solution comprenant un agent antimicrobien comprenant de l'argent, ou un sel pharmacologiquement acceptable de celui-ci, et un oxyde, ledit oxyde étant choisi dans le groupe constitué par l'oxyde de cuivre, l'oxyde de fer, l'oxyde de zinc, le dioxyde de titane et le dioxyde de silice ; et
- laisser sécher le couvercle (3) ;
ledit couvercle (3) étant au moins partiellement rempli, ou adapté pour être au moins partiellement rempli, d'un moyen de régulation de température (40), et, lors de l'utilisation, ledit couvercle (3) étant agencé de manière à entrer en contact avec l'œil d'un sujet et/ou une zone environnante de celui-ci, et ledit moyen de régulation de température (40) étant conçu pour moduler la température du couvercle (3) de manière à ainsi traiter, prévenir ou améliorer l'état oculaire.

14. Procédé selon la revendication 13, ledit fait de laisser sécher le couvercle (3) comprenant le chauffage du couvercle (3) à une température située entre 30°C et 150°C, ou entre 40°C et 100°C, ou entre 50°C et 80°C, ou entre 55°C et 65°C pendant une durée prédéfinie, éventuellement ladite durée prédéfinie étant d'au moins 5 minutes, ou d'au moins 30 ou 60 minutes, ou d'au moins 1 heure 30 minutes.

15. Kit destiné à fabriquer un dispositif médical (2), le kit comprenant
- un dispositif comprenant un couvercle (3) qui est au moins partiellement rempli d'un moyen de régulation de température (40) ; et
- une solution comprenant un agent antimicrobien conçue pour être appliquée sur le couvercle, ledit agent antimicrobien comprenant de l'argent, ou un sel pharmacologiquement acceptable de celui-ci, et un oxyde, ledit oxyde étant choisi dans le groupe constitué par l'oxyde de cuivre, l'oxyde de fer, l'oxyde de zinc, le dioxyde de titane et le dioxyde de silice ;
ledit couvercle (3) étant agencé, lors de l'utilisation, de manière à entrer en contact avec l'œil d'un sujet et/ou une zone environnante de celui-ci, et ledit moyen de régulation de température (40) étant conçu pour moduler la température du couvercle (3) de manière à ainsi traiter, prévenir ou améliorer un état oculaire.
